# EUROPEAN PATENT SPECIFICATION

(11) **EP 2 376 186 B1**
(45) Date of publication and mention of the grant of the patent: **30.03.2016**
(21) Application number: 09752263.5
(22) Date of filing: 11.11.2009
(51) Int. Cl.: A61N 1/36, A61N 1/378, A61N 1/375, H04R 25/00

(54) **INTEGRATED COCHLEAR IMPLANT HEADPIECE**
INTEGRIERTES COCHLEAIMPLANTAT-KOPFTEIL
OREILLETTE INTÉGRÉE POUR IMPLANT COCHLÉAIRE

(30) Priority: 04.03.2009 US 398058; 12.11.2008 US 113675 P; 20.12.2008 US 139567 P
(43) Date of publication of application: 19.10.2011
(73) Proprietor: Advanced Bionics AG, 8712 Stäfa (CH)
(72) Inventor: CRAWFORD, Scott, A., Castaic CA 91384 (US); LYNCH, Douglas, P., Shepherdstown WV 25443 (US); WOODS, Carla, Mann, Beverly Hills CA 90210 (US); GRIFFITH, Glen, A., Newbury Park CA 91320 (US)
(74) Representative: Schwan Schorer & Partner mbB
(86) International application number: PCT/US2009/064068
(87) International publication number: WO 2010/056770

(56) References cited:
- WO-A-2005/062668
- US-A- 5 949 895
- US-A1- 2002 032 472
- US-A1- 2004 073 275
- US-A1- 2004 133 065
- US-A1- 2005 245 991
- US-A1- 2006 015 155
- US-A1- 2006 190 059
- US-A1- 2007 053 534
- US-A1- 2007 191 673
- US-A1- 2007 282 394
- US-A1- 2008 002 834
- US-B1- 6 246 911

## Description

### BACKGROUND

In human hearing, hair cells in the cochlea respond to sound waves and produce corresponding auditory nerve impulses. These nerve impulses are then conducted to the brain and perceived as sound.

Hearing loss, which may be due to many different causes, is generally of two types: conductive and sensorineural. Conductive hearing loss typically occurs where the normal mechanical pathways for sound to reach the hair cells in the cochlea are impeded, for example, from damage to the ossicles. Conductive hearing loss may often be helped by using conventional hearing aids that amplify sounds so that acoustic information can reach the cochlea and the hair cells. Some types of conductive hearing loss are also amenable to alleviation by surgical procedures.

Many people who are profoundly deaf, however, have sensorineural hearing loss. This type of hearing loss can arise from the absence or the destruction of the hair cells in the cochlea which then no longer transduce acoustic signals into auditory nerve impulses. Individuals with sensorineural hearing loss may be unable to derive any meaningful benefit from conventional hearing aid systems no matter how loud the acoustic stimulus is. This is because the mechanism for transducing sound energy into auditory nerve impulses has been damaged. Thus, in the absence of properly functioning hair cells, auditory nerve impulses cannot be generated directly from sounds.

To overcome sensorineural deafness, cochlear implant systems or cochlear prostheses have been developed that can bypass the hair cells located in the cochlea by presenting electrical stimulation directly to the auditory nerve fibers. This leads to the perception of sound in the brain and provides at least partial restoration of hearing function. Most of these cochlear prosthesis systems treat sensorineural deficit by stimulating the ganglion cells in the cochlea directly using an implanted electrode or lead that has an electrode array. Thus, a cochlear prosthesis operates by directly stimulating the auditory nerve cells, bypassing the defective cochlear hair cells that normally transduce acoustic energy into electrical activity to the connected auditory nerve cells.

Prior to stimulating the nerve cells, the electronic circuitry and the electrode array of the cochlear prosthesis separate acoustic signals into a number of parallel channels of information, each representing a narrow band of frequencies within the perceived audio spectrum. Ideally, each channel of information should be conveyed selectively to a subset of auditory nerve cells that normally transmit information about that frequency band to the brain. Those nerve cells are arranged in an orderly tonotopic sequence, from the highest frequencies at the basal end of the cochlear spiral to progressively lower frequencies towards the apex.

A cochlear implant system typically comprises both an external unit that receives and processes ambient sound waves and a cochlear implant that receives data from the external unit and uses that data to directly stimulate the auditory nerve. A common configuration for a cochlear implant system thus involves internal components that are surgically implanted into the patient and external components that provide power and electrical signals representing environmental sound to the internal components. These external components typically include a Behind-the-Ear (BTE) processor worn on the ear or a body worn processor. These processors contain a microphone, batteries, and signal circuitry that processes the electrical signals generated by the microphone. The processors are connected to a headpiece by a cable. The headpiece receives the electrical signals through the cable and transmits them to the internal components.

In some cochlear implant systems, the cable or cables connecting the external components together can present some issues. For example, the cable may have to be routed through clothing or accommodated during hair styling. The cable may be snagged, pulled on, or tangled, causing the headpiece to fall off. Additionally, cables are considered unattractive by many patients and are susceptible to failure due to bending.

US 2006/0190059 A1 and US 2007/053534 A1 relate to behind-the-ear (BTE) sound processors with an integral induction coil for transmitting a processed audio signal to a cochlea implant. US 5,949,895 relates to an external sound processor for implantable hearing devices including a microphone, signal processing electronics and a wireless transmitter for the implanted receiver; the sound processor is designed as a headpiece which is to be fixed at the head at a position behind the patient's ear. Similar devices are described in US 2008/002834 A1 and US 2004/133065 A1. US 2002/0032472 A1 relates to an implanted device including a circuit board housed within a cage which is formed from material having high magnetic permeability and a secondary coil. The implanted device receives energy via transcutaneous energy transfer from an external primary coil. The cage reduces inductive heating of the components on the circuit board and includes a flange which is located adjacent to the secondary coil and guides magnetic flux lines back to the external primary coil.

### BRIEF DESCRIPTION OF THE DRAWINGS

The accompanying drawings illustrate various embodiments of the principles described herein and are a part of the specification. The illustrated embodiments are merely examples and do not limit the scope of the claims.
FIG. 1 illustrates an exemplary cochlear implant system according to principles described herein.
FIG. 2 is a functional block diagram of an exemplary sound processor and implantable cochlear stimulator according to principles described herein.
FIG. 3 illustrates a schematic structure of the human cochlea highlighting elements according to principles described herein.
FIG. 4A is an exploded diagram of various illustrative components which may make up an exemplary integrated headpiece.
FIG. 4B is a side view of an exemplary integrated headpiece.
FIG. 5 is a diagram showing the internal components of an illustrative cochlear implant system.
FIG. 6 is a perspective view of an exemplary integrated headpiece.
FIG. 7A is a diagram showing an illustrative integrated headpiece being worn by a user.
FIG. 7B is an illustrative diagram showing an illustrative integrated speech processor headpiece interfacing with internal components of a cochlear implant system.
FIG. 8 is a perspective view of an illustrative integrated headpiece.
FIG. 9 is a top view of an illustrative integrated headpiece.
FIG. 10 is a perspective view of an illustrative integrated headpiece.
FIG. 11A is an exploded perspective view of an integrated speech processor headpiece according to principles of the present invention.
FIG. 11 B is a cross-sectional side view of a portion of an integrated speech processor headpiece according to principles of the present invention.
FIG. 12 illustrates magnetic flux surrounding an induction coil and a retention magnet in an integrated speech processor headpiece according to principles of the present invention.

Throughout the drawings, identical reference numbers designate similar, but not necessarily identical, elements.

### DETAILED DESCRIPTION

As mentioned above, individuals with hearing loss can be assisted by a number of hearing assistance devices. These assistive devices are typically worn regularly and over a significant period of each day. Consequently, any such hearing assistance device should be robust and reliable. Additionally, the hearing assistance devices should be visually unobtrusive and not unduly restrict the user's activities. As explained above, cochlear implant users typically must wear at least two separate external units, a processor and a headpiece, that are connected by a cable.

The processor in a conventional system may be a Behind-The-Ear (BTE) processor or a body worn processor. A BTE processor typically uses a hook which attaches over the top of the outer ear and holds the BTE processor in place behind the ear of the user. The BTE processor contains a microphone, battery, and electronics. A cable attaches the BTE processor to the headpiece and conveys data signals and power to the headpiece. The headpiece is typically held in place by magnetic forces generated by a surgically implanted magnet that is a part of the internal cochlear implant.

A body worn processor is typically worn by attaching the processor to an article of clothing worn by the user. For example, a body worn processor may be tucked into a pocket or attached to a lapel. The body worn processor does not have the severe size and weight constraints that are associated with a BTE processor. Consequently, the electronics and battery capacity of the body worn processor can be significantly greater than BTE processors. Like the BTE processor, a cable attaches the body worn processor to the headpiece.

As mentioned above, the cable or cables connecting the external components together can be difficult to manage. For example, when a child wears a cochlear implant, the parent may have to take additional care in dressing the child and restrict some activities the child would otherwise enjoy to prevent the cable from being snagged, pulled on, tangled, or broken. Additionally, the processor and cable can be visually distracting and are considered unattractive by many patients. For some patients, the BTE unit can be uncomfortable, particularly those who are sensitive to heavy objects hanging from their ears.

The invention relates to an integrated headpiece for a cochlear implant system as defined in claim 1 and a system of integrated headpieces for a cochlear implant system as defined in claim 8.

Such integrated cochlear implant headpiece combines the external components of the cochlear system into a single unit that is worn directly over the surgically implanted receiver. The integrated cochlear implant headpiece is a head mounted, external component unit which provides a stand-alone support for the functionalities of the implanted components. This eliminates the need for a separate body worn processor or BTE processor and the connecting cable. Consequently, the integrated cochlear implant headpiece reduces the difficulties commonly associated with wearing and using a cochlear implant. Specifically, because there is no separate processor unit or connecting cable, there is no need to route a cable through clothing or hair and no possibility of snagging or damaging the cable. Additionally, the integrated cochlear implant headpiece can be significantly less visually intrusive and more user friendly.

In some embodiments, the integrated cochlear implant headpiece may have one or more accessories which attach to the integrated headpiece and provide additional functionality. As discussed in U.S. Patent No. 7,599,508, an assistive listening cap may magnetically attach to the top of the integrated cochlear implant headpiece. The assistive listening device may provide a variety of benefits to the patient. By way of example and not limitation, the assistive listening device may provide additional battery power; alternative antennas and circuitry for receiving audio signals via electromagnetic transmission; additional memory capacity; and/or additional signal processing capability.

In the following description, for purposes of explanation, numerous specific details are set forth in order to provide a thorough understanding of the present systems and methods. It will be apparent, however, to one skilled in the art that the present systems and methods may be practiced without these specific details. Reference in the specification to "an embodiment," "an example," or similar language means that a particular feature, structure, or characteristic described in connection with the embodiment or example is included in at least that one embodiment, but not necessarily in other embodiments. The various instances of the phrase "in one embodiment" or similar phrases in various places in the specification are not necessarily all referring to the same embodiment. Also, as used in the specification and appended claims the term "headpiece" refers to a component that is worn on the user's head in proximity to an internal antenna, as opposed to a BTE processor or a body worn processor that are worn on the ear or on an article of clothing.

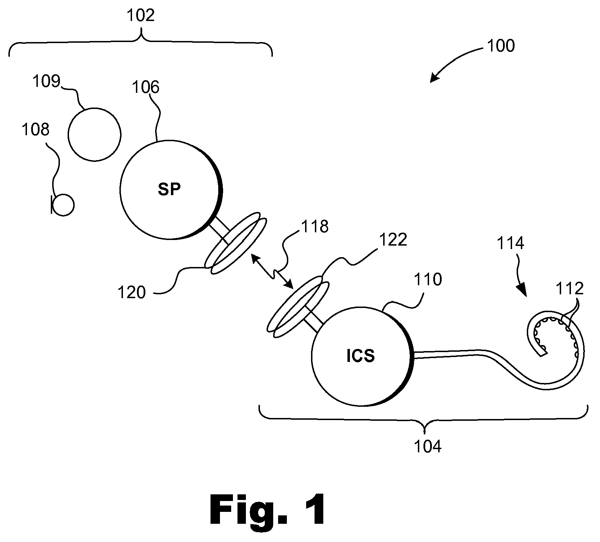

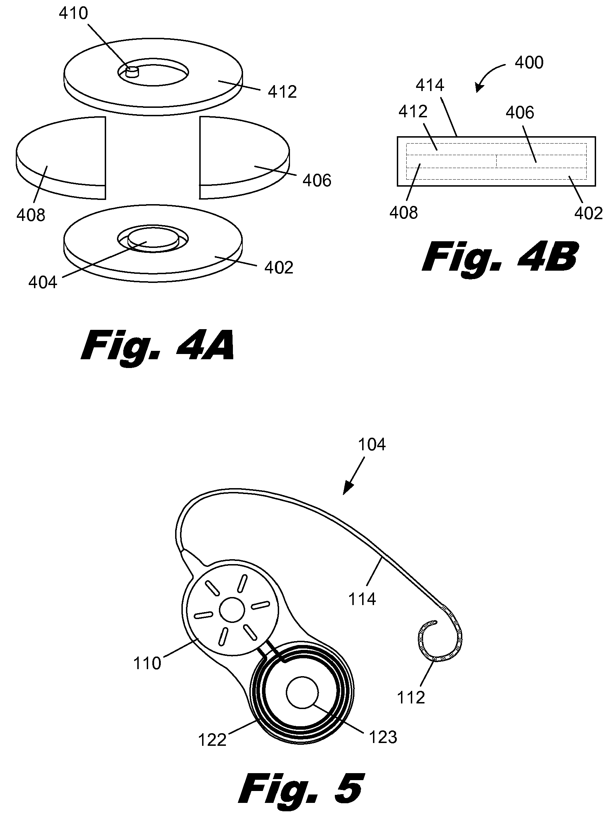

FIG. 1 illustrates an exemplary cochlear implant system 100. The cochlear implant system 100 of FIG. 1 includes an integrated speech processor headpiece (or "integrated headpiece") 102 and a cochlear stimulation device 104. The integrated headpiece 102 may include a sound processor 106, a microphone 108, one or more batteries 109, a communication device such as the illustrated induction coil 120, and/or additional circuitry and instrumentalities as best serves a particular application. As discussed below, the integrated headpiece 102 consolidates all of the external components of the cochlear implant system in one compact unit. This eliminates cables connecting the traditional components together and the associated problems of routing the cables through clothing or the cable being snagged, pulled on, or tangled, causing the headpiece to fall off. The cochlear stimulation device 104 may include an implantable cochlear stimulator 110, a number of electrodes 112 disposed on an electrode lead 114, a communication device such as the illustrated induction coil 122, and/or additional circuitry as best serves a particular application. The components within the sound processor portion 102 and the cochlear stimulation device 104 will be described in more detail below. For example, the integrated aspects of the present systems are discussed below with reference to FIGS. 4A-10. Moreover, detailed descriptions of various aspects of cochlear implant systems that may be applicable to the present inventions, but are not necessary for an understanding of the present inventions, are disclosed in U.S. Patent Nos. 6,219,580; 6,272,382; and 6,308,101.

The microphone 108 of the integrated headpiece 102 is configured to sense audio signals and convert the sensed signals to corresponding electrical signals. In some examples, the audio signal may include speech. The audio signal may additionally or alternatively include music, noise, and/or other sounds. The electrical signals are sent from the microphone 108 to the sound processor 106. The microphone 108 may be integrated into the integrated headpiece 102. Alternatively, the microphone 108 may be connected to the integrated headpiece 102 by way of a communication link or a cable. The sound processor 106 processes these converted audio signals from the microphone in accordance with a selected sound processing strategy to generate appropriate stimulation parameters for controlling the implantable cochlear stimulator 110. These stimulation parameters may specify or define the polarity, magnitude, location (i.e., which electrode pair or electrode group receive the electrical stimulation), stimulation rate, timing (i.e., when the electrical stimulation is to be applied to a particular electrode pair), spectral tilt, and/or any other characteristic of the electrical stimulation that is generated by the implantable cochlear stimulator 110. In some alternative embodiments, one or more portions of the sound processor 106 may be included within the implantable portion (e.g. cochlear stimulation device 104) of a cochlear implant system.

The exemplary electrode lead 114 shown in FIG. 1 is configured to be inserted within a duct of a cochlea. The electrode lead 114 includes a multiplicity of electrodes 112, for example, sixteen electrodes, spaced along its length. It will be understood, however, that any number of electrodes 112 may be disposed on the electrode lead 114. The electrode lead 114 may be substantially as shown and described in U.S. Patent Nos. 4,819,647 or 6,218,753.

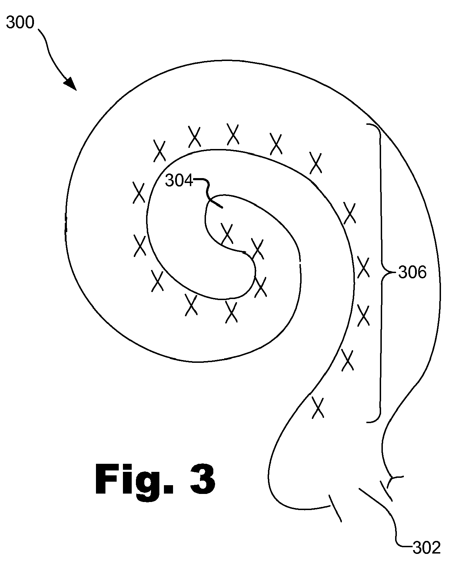

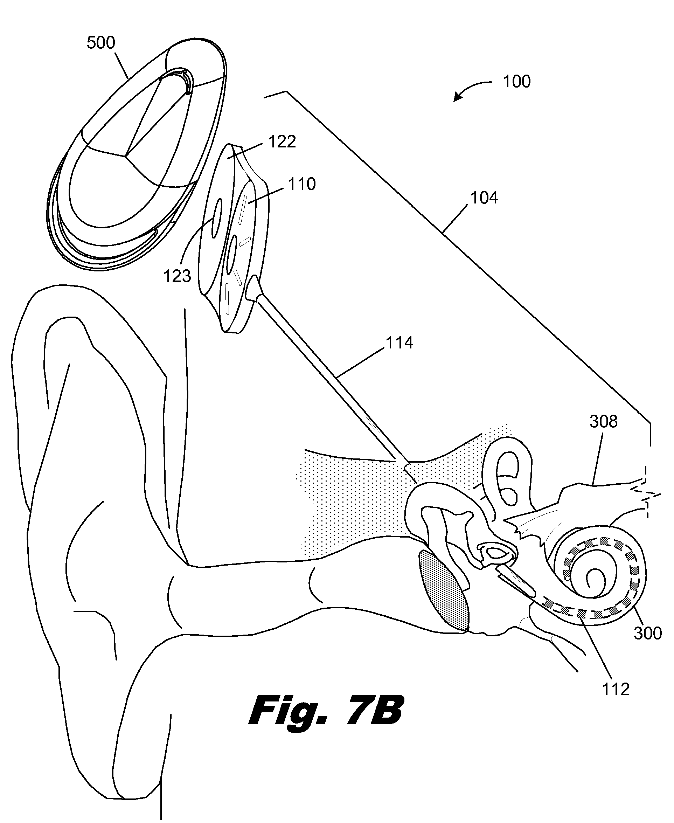

As will be described in more detail below, and referring to FIGS. 3 and 7B, electronic circuitry within the implantable cochlear stimulator 110 is configured to generate and apply electrical stimulation to one or more stimulation sites within the cochlea 300 via selected stimulation channels (i.e., pairs or groups of the individual electrodes 112) in accordance with a specified stimulation strategy defined by the sound processor 106, and neural electrical signals are conducted from the cochlea 300 to the auditory cortex by the auditory nerve 308.

The exemplary implantable cochlear stimulator 110 and the sound processor 106 may be communicatively coupled via a suitable data or communication link 118, such as a telemetry communication link, as will be described in more detail below. It will be understood that the data communication link 118 may include a bi-directional communication link and/or one or more dedicated uni-directional communication links. In some examples, the external and implantable portions of the cochlear implant system 100 may each include one or more inductive coils (discussed below) configured to transmit and receive power and/or control signals via the communication link 118. The control signals may include, for example, the magnitude and polarity of electrical stimulation representing a sensed audio signal. The external coil may also transmit power from the external portion to the implantable portion of the cochlear implant system 100. Power transmitted to the implantable portion may be used to operate the implantable portion.

In the exemplary cochlear implant system 100, the integrated speech processor headpiece 102 may include an external induction coil 120 and the cochlear stimulation device 104 may include an implantable induction coil 122. The external induction coil 120 may be communicatively coupled to the sound processor 106 and the implantable induction coil 122 may be communicatively coupled to the implantable cochlear stimulator 110. It should be noted here that the present inventions are not limited to electromagnetic induction based communication devices. Other exemplary communication devices includes, but are not limited to, radio frequency communication devices, optical communication devices, and any other wireless communication devices.

The external induction coil 120 and the implantable induction coil 122 may include any suitable type of coil capable of generating and/or receiving an electro-magnetic field. For example, the external induction coil 120 and the implantable induction coil 122 may each include a metallic wire or tube wound in a coiled or otherwise looped configuration. An alternating current may be directed from the sound processor 106 through the external induction coil 120, thereby generating a magnetic field surrounding the external induction coil 120. The external induction coil 120 may be positioned near the implantable induction coil 122 such that the implantable induction coil 122 is positioned at least partially within the magnetic field generated by the external induction coil 120. The magnetic field generated by the external induction coil 120 may cause an electric current to be generated in the internal induction coil 120. The electric current generated in the internal induction coil 120 may be directed from the internal induction coil 120 to the implantable cochlear stimulator 110. Accordingly, an electric current generated by the sound processor 106 may be transferred to the implantable cochlear stimulator 110 through the communication link 118 comprising the external induction coil 120 and the implantable induction coil 122.

The communication link 118 may function as a telemetry link between the sound processor 106 and the implantable cochlear stimulator 110. For example, the external induction coil 120 may transmit one or more telemetry signals to the implantable induction coil 122 by generating a telemetry magnetic field as electric current is passed through the external induction coil 120. The telemetry magnetic field generated by the external induction coil 120 may produce an electric current in the implantable induction coil 122, as described above. The current generated in the implantable induction coil 122 by the telemetry magnetic field generated by the external induction coil 120 may be used to transfer signals representative of data and/or other information to the implantable cochlear stimulator 110 and/or may be used to transfer power to the implantable cochlear stimulator 110.

In some examples, the communication link 118 may be used to transmit telemetry signals from the implantable cochlear stimulator 110 to the sound processor 106. For example, data acquired by the electrodes 112 and/or status indicators generated by the cochlear stimulator 112 may be transmitted to sound processor 106 via the communication link 118. To this end, implantable induction coil 122 may transmit telemetry signals to the external induction coil 120 by generating a telemetry magnetic field. The implantable cochlear stimulator 110 may cause a current to flow through the implantable induction coil 122 to generate the telemetry magnetic field. The external induction coil 120 may be positioned at least partially within the telemetry magnetic field generated by the implantable induction coil 122. The magnetic field may cause an electric current to be generated in the external induction coil 120. The current generated in the external induction coil 120 may be used to transfer data and/or other signals to the sound processor 106.

The communication link 118 may include more than one external induction coil 120 and/or more than one implantable induction coil 122. For example, a first external induction coil and a first implantable induction coil may be used to transfer data and/or power from the sound processor 106 to the implantable cochlear stimulator 110. A second external induction coil and a second implantable induction coil may be used to transfer data from the implantable cochlear stimulator 110 to the sound processor 106.

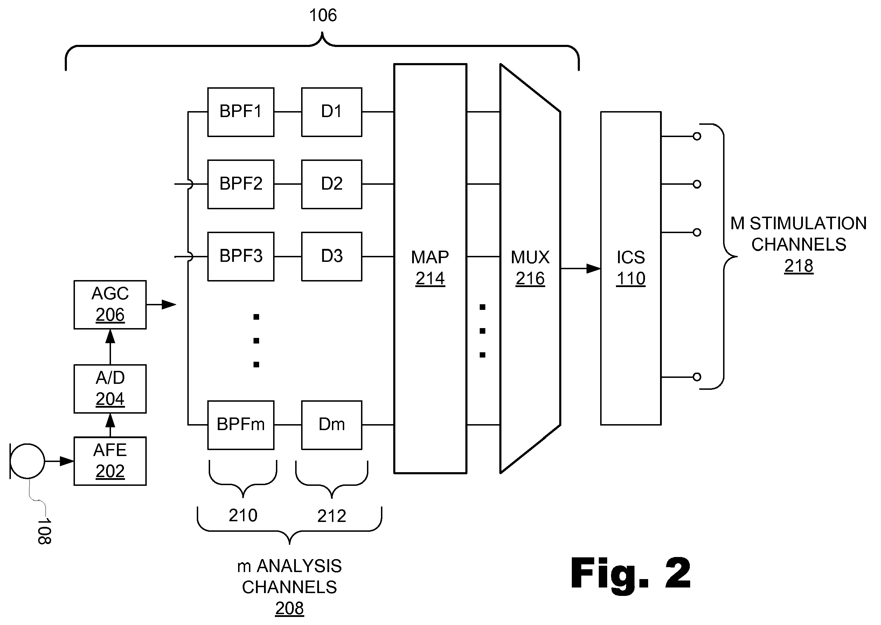

FIG. 2 is a functional block diagram of an exemplary sound processor 106 and implantable cochlear stimulator 110. The functions shown in FIG. 2 are merely representative of the many different functions that may be performed by the sound processor 106 and/or the implantable cochlear stimulator 110.

As shown in FIG. 2, the microphone 108 senses an audio signal, such as speech or music, and converts the audio signal into one or more electrical signals. These signals are then amplified in audio front-end (AFE) circuitry 202. The amplified audio signal is then converted to a digital signal by an analog-to-digital (A/D) converter 204. The resulting digital signal is then subjected to automatic gain control using a suitable automatic gain control (AGC) function 206.

After appropriate automatic gain control, the digital signal is then processed in one of a number of digital signal processing or analysis channels 208. For example, the sound processor 106 may include, but is not limited to, sixteen analysis channels 208. Each analysis channel 208 may respond to a different frequency band of the sensed audio signal due to a series of band pass filters 210.

As shown in FIG. 2, each of the m analysis channels 208 may also include an energy detection stage (D1-Dm) 212. Each energy detection stage 212 may include any combination of circuitry configured to detect the amount of energy contained within each of the m analysis channels 208. For example, each energy detection stage 212 may include a rectification circuit followed by an integrator circuit.

After energy detection, the signals within each of the m analysis channels 208 are forwarded to a mapping stage 214. The mapping stage 214 is configured to map the signals in each of the m analysis channels 208 to one or more of M stimulation channels 218. In other words, the information contained in the m analysis channels 208 is used to define the electrical stimulation pulses that are applied to the patient by the implantable cochlear stimulator 110 via the M stimulation channels 218. As mentioned previously, pairs or groups of individual electrodes 112 may make up the M stimulation channels 218.

In some examples, the mapped signals are serialized by a multiplexer 216 and transmitted to the implantable cochlear stimulator 110. The implantable cochlear stimulator 110 may then apply electrical stimulation via one or more of the M stimulation channels 218 to one or more stimulation sites within the duct of the patient's cochlea. As used herein, the term "stimulation site" will be used to refer to a target area or location to which the electrical stimulation is applied. For example, a stimulation site may refer to any location within a region of auditory nerve tissue.

FIG. 3 illustrates a schematic structure of the human cochlea 300. As shown in FIG. 3, the cochlea 300 is in the shape of a spiral beginning at a base 302 and ending at an apex 304. Within the cochlea 300 resides auditory nerve tissue 306, which is denoted by Xs in FIG. 3. The auditory nerve tissue 306 is organized within the cochlea 300 in a tonotopic manner. Low frequencies are encoded at the apex 304 of the cochlea 300 while high frequencies are encoded at the base 302. Hence, each location along the length of the cochlea 300 corresponds to a different perceived frequency. A cochlear prosthesis may therefore be implanted within a patient with sensorineural hearing loss and configured to apply electrical stimulation to different locations within the cochlea 300 to provide the sensation of hearing. For example, electrode lead 114 may be disposed within the cochlea 300 such that electrodes 112 contact auditory nerve tissue 306 within the cochlea 300. Electrical stimulation may be applied by the electrodes 112 to the auditory nerve tissue 306.

FIGS. 4A and 4B show one exemplary manner of incorporating various components into an integrated headpiece 400. Referring first to FIG. 4A, the integrated headpiece 400 may include an induction coil 402 or other transmitter antenna, a retention magnet 404, a battery 406, electronics for audio signal processing 408, and a microphone 410. Headpiece 400 may also optionally include a receiver 412 for receiving signals from an external source. Each of these components is discussed below. The components illustrated in FIG. 4A are carried within or by the housing 414 illustrated in FIG. 4B. In other words, the integrated headpiece 400 consolidates all of the external components of the cochlear implant system in one compact unit. This eliminates cables connecting the traditional components together and the associated problems of routing the cables through clothing or the cable being snagged, pulled on, or tangled, causing the headpiece to fall off. Additionally, the integrated headpiece 400 may be more discrete than systems with multiple components. For example, the integrated headpiece 400 may be completely covered by the user's hair or hat. Further, the integrated headpiece 400 may be more robust than multiple component configurations. The integrated headpiece 400 may be much easier to seal because there is no need for external connection or cables.

The induction coil 402 transmits signals to the implanted coil 122 associated with the exemplary cochlear stimulation device 104 (FIG. 5). According to one embodiment, the induction coil 402 also inductively transmits power to the cochlear stimulation device 104 or other internal components. The magnet 404 in the center of the induction coil 402 is attracted to an implanted magnetic 123 that is in the center of the implanted antenna 122. The attraction between the magnet 404 and the implanted magnetic 123 holds the integrated headpiece 400 over the antenna 122. The induction coil 402 may also be used to receive power to charge the battery 406 when the integrated headpiece 400 is not in use. For example, the induction coil 402 could be used to inductively charge the battery by placing in the induction coil in proximity to a charging coil through which an alternating current is passed. The induction coil 122 acts as a transformer coil and receives a portion of the energy. This energy can then be used to charge the battery within the integrated headpiece. One advantage of using inductive coupling to charge batteries is that the headpiece can be more easily sealed because there is no need for exposed conductors or connectors.

The magnet 404 may be made from any of a number of magnetic materials including, but not limited to, neodymium-iron-boron, samarium-cobalt, ticonal, alnico, ceramic, magnetic powder in a resin matrix, or other suitable material. According to one embodiment, materials which exhibit a higher magnetic strength per unit volume may be used to minimize the size of the magnet and integrated headpiece 400.

The battery 406 supplies electrical energy that is required for the function of the cochlear implant. Important considerations for a battery included in the integrated headpiece may include the energy density, total capacity of the battery, voltage, robustness, the ability to hold a charge over a long period of time, and the ability to be repeatedly charged and discharged.

By way of example and not limitation, the battery may a lithium ion battery, a polymer lithium battery, a zinc air battery or other suitable battery. Polymer lithium batteries operate using the same chemistry as conventional lithium ion batteries but contain the lithium-salt electrolyte within a solid polymer composite rather than a rigid metal case. Consequently, polymer lithium batteries can be lighter, more energy dense, and less vulnerable to physical damage. Further, polymer lithium batteries can be specifically shaped to fit the device it will power. Zinc air batteries operate by the oxidation of zinc with atmospheric oxygen. Zinc air batteries have high energy densities and are relatively inexpensive to produce. However, to operate, zinc air batteries must have direct exposure to the atmosphere, which creates challenges in using these batteries in sealed systems.

The electronics 408 may include components and functionality such as power conditioning electronics, signal processors, filters, amplifiers, receivers, switches, memory, and other electronics. The principal function of the electronics 408 is to receive an audio signal from the microphone 410 and process that signal into a signal that can be transmitted to the implanted unit to drive stimulation of the cochlea.

A number of additional components may be included in the integrated headpiece. For example, various visual indicators, such as one or more light emitting diodes, could also be included. These visual indicators could be configured to communicate information regarding the function of both internal and external components of the cochlear implant system, such as battery status, the selected program, sensitivity or volume information, and communication status between the headpiece and implanted receiver.

The integrated headpiece may optionally include a receiver 412. The receiver 412 may be any one of a variety of radio frequency (RF), WiFi, IEEE 802.11, Bluetooth®, or other receivers. These receivers can directly link the cochlear implant system to sound sources, reducing undesirable interference by other noise sources. The sound sources may include a wireless microphone, a remote control device, a cell phone, a computer, a music player, a personal digital assistant, or other device. For example, in an educational setting, teacher may wear a wireless microphone which transmits the teacher's voice over a radio frequency directly to a receiver contained within the integrated headpiece. Similarly, a Bluetooth® receiver could be connected to a stereo, cell phone, or other audio source.

A microphone 410 is also included within the integrated headpiece. The microphone 410 may reside directly on the electronics component or may be a separate component that sends electrical signals through a wired connection to the electronics. A variety of microphone types and configurations may be used. By way of example and not limitation, the microphone may use electromagnetic, capacitance change, MicroElectroMechanical Systems (MEMS) or piezoelectric mechanisms to produce an electrical signal from mechanical vibrations induced by sound. The microphone may also have one of many directional sensitivity profiles. For example, the microphone may have an omnidirectional, hemispherical, subcardioid, cardioid, or highly directional sensitivity profile.

The components described above with reference to FIGS. 1-4B can be configured in a number of different shapes and contained with a variety of housings to form an integrated cochlear implant headpiece.

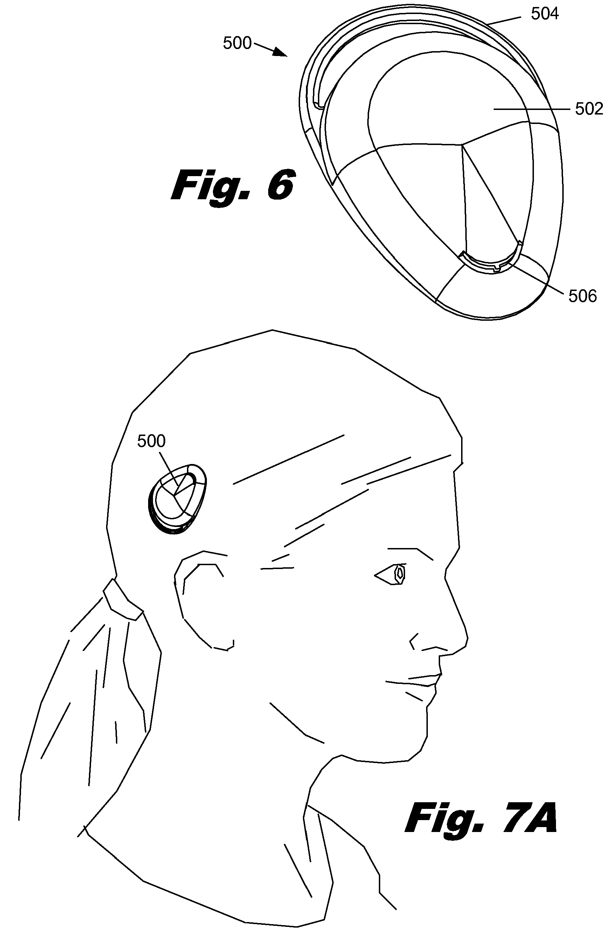

One exemplary configuration of an integrated headpiece is generally represented by reference numeral 500 in FIG. 6. In this illustrative embodiment, a body portion (or "housing") 502 of the integrated headpiece 500 contains a battery, electronics, a microphone, and a magnet, such as those described above.

A portion of the transmitter antenna 504 extends beyond the body portion 502, forming an open loop. This transmitter antenna configuration may have a number of advantages that extend beyond the visual appearance of the headpiece 500. For example, the transmitter antenna 504 may additionally serve as an inductive pick up which receives electrical energy from an exterior coil to charge the internal battery.

The exposed portion of the transmitter antenna 504 may also allow for more efficient transfer of the electrical power from the exterior coil because the exterior coil could substantially surround the transmitter antenna. In one charging configuration, the exposed portion of the transmitter antenna 504 may be inserted into a corresponding slot in a charging platform. One or more charging coils could be placed on either side of the slot. The transmitter antenna 504 would then be in very close proximity to the charging coils. Additionally, the exposed portion transmitter antenna 504 could assist in the proper positioning and retention of the integrated headpiece 500 within the charging platform.

The integrated headpiece may also have a number of other features. As mentioned above, visual indicators could be incorporated into the external shell of the integrated headpiece to allow a caretaker to visually ascertain the state and functionality of the integrated headpiece. By way of example and not limitation, the indicating features may include light emitting diodes which indicate the battery condition. For example, as the battery discharges, a light emitting diode is illuminated to indicate the need to recharge or replace the battery. This could be advantageous for a parent or teacher who can visually determine the battery level.

Similarly, the integrated headpiece may have one or more visual elements which indicate the state of the cochlear implant. For example, a light emitting diode could have a first color and illumination pattern which indicates that cochlear implant is operational. The light emitting diode could have a different color and/or illumination pattern for various malfunctions such as a malfunction of the integrated headpiece, lack of communication between the integrated headpiece and implanted receiver, or a receiver malfunction.

In the illustrative embodiment shown in FIG. 6, a switch 506 is included to allow the user to adjust the amplification of the device or to switch between a number of predetermined programs. For example, a first program may be specially adapted for personal conversations with minimal background noise, while a second program may be optimized for environments with much higher levels of background noise, such as a restaurant or crowded convention venue. A third program setting may activate a Radio Frequency (RF) receiver that is linked to a microphone worn by a teacher, lecturer, or conversationalist. By including external switches, the user can adjust the integrated headpiece in real time to suit a particular sound environment.

FIG. 7A shows the exemplary integrated headpiece 500 being worn by a user, while FIG. 7B shows the exemplary integrated headpiece 500 interfacing with internal components of a cochlear implant system 100, e.g. the above-described cochlear stimulation device 104. As discussed above, an integrated headpiece is worn over the antenna implantation site. The antenna is typically implanted above and behind the external ear as shown in FIGS. 7A and 7B. However, the antenna may be implanted in a variety of other locations.

If desired, the user can conceal the integrated headpiece 500 by altering her hair style or wearing a head covering such as a hat, hood, or scarf. If the user has access to a variety of integrated headpieces, the user can select the integrated headpiece that is most suited for a given activity or day. The user may carry one or more backup headpieces in a pocket, purse, or backpack. If circumstances during the day make it desirable to replace the current integrated headpiece with an alternative headpiece, the user can simply reach up, grasp the integrated headpiece 500 and remove it. A second integrated headpiece is then retrieved, oriented with the magnet side toward the head, and brought to the approximate location of the implanted antenna. As the second integrated headpiece nears the antenna location, the magnetic attraction between the two magnetic components moves the integrated headpiece into the correct location and holds the integrated headpiece in place.

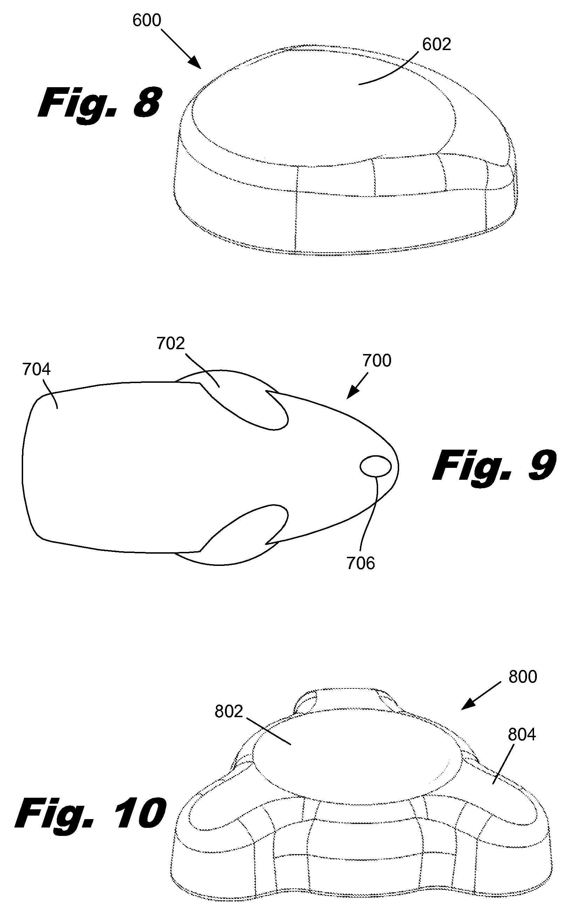

FIG. 8 is a perspective view of an illustrative integrated headpiece 600. In this illustrative example, the exterior profile of the headpiece 600 is simplified, which may result in a more robust and inexpensive device. The headpiece 600 includes a common rigid housing 602 that contains a microphone, signal processing electronics, transmitter and battery or other power supply, such as those described above. The rigid housing maintains its shape during normal handling and provides protection for the internal components against external contaminants and impact. Additionally, external switches and visual indicators may be omitted from the headpiece. By eliminating these external features, the headpiece may be more easily constructed to be waterproof, allowing the user to walk in the rain, swim, or participate in water sports without removing the headpiece. This will allow the user to continue to receive auditory signals, thereby enhancing the user's enjoyment of the activity, ability to interact with others, and increasing the user's safety.

The headpiece may be linked via a receiver in the headpiece to an external control unit. For example, a number of controls could be incorporated into a key fob. By pressing buttons on the key fob, wireless signals could be sent to adjust the operational parameters of the integrated headpiece.

FIG. 9 is a top view of an illustrative integrated headpiece 700 with an alternative geometry. In this illustrative example, the transmitter antenna may be contained within a center portion 702 of the integrated headpiece 700. Other components may be contained within the body portion 704. According to one illustrative embodiment, the integrated headpiece 700 may additionally include a directional microphone 706. Typically, omnidirectional or hemispherical microphones are used in cochlear devices to better replicate the sensitivity of the human ear. However, in some circumstance it may be beneficial to more selectively sense external sounds, thereby reducing background noise. The directional microphone 706 can be used by the patient to selectively amplify selected sound sources. According to one embodiment, the directional microphone 706 may be pointing in the same direction the patient is looking. For example, a patient may simply turn his head toward one who is speaking to point the directional microphone 706 in the speaker's direction to preferentially sense his voice.

The lower cost and ease of wearing the integrated headpiece can lead to a number of benefits. For example, the patient may have two or more integrated headpieces. While one integrated headpiece is being worn by the user, the other integrated headpiece can be recharging its battery.

Additionally or alternatively, the functionality provided by a second integrated headpiece may be different. The user can then select the integrated headpiece that is most appropriate for the situation. For example, during a social event, the user may select an integrated headpiece that is less obtrusive or complements other clothing accessories. During the course of a normal day, the user may select an integrated headpiece with a longer lifetime or with a needed receiver. For example, if the user attends school, the user may need a battery that can supply power throughout the school day and a receiver that can receive amplified/filtered signals from a wireless microphone worn by the teacher. If the user is participating in water activities, a sealed headpiece could be selected.

FIG. 10 is a perspective view of an illustrative integrated headpiece 800 with an alternative geometry. The integrated headpiece 800 has a housing with a central body portion 802 and a number of lobes 804 which surround the central body portion. The housing contains a microphone, signal processing electronics, transmitter and battery or other power supply, such as those described above, in the central body portion 802 and/or lobes 804. These lobes 804 may serve a number purposes including increasing the stability of the headpiece, creating a visually interesting profile, increasing the internal volume of the integrated headpiece 800, providing a shape that is adapted to a particular transmitter or receiver, or covering a lithium polymer battery which is particularly shaped to be received by the lobes 804.

According to the invention, the present integrated speech processor headpiece is configured to direct magnetic flux associated with the telemetry magnetic field generated by the telemetry coil (e.g. coil 102 or coil 402) and the retention magnetic field generated by a retention magnet (e.g. magnet 404) away from certain circuitry.

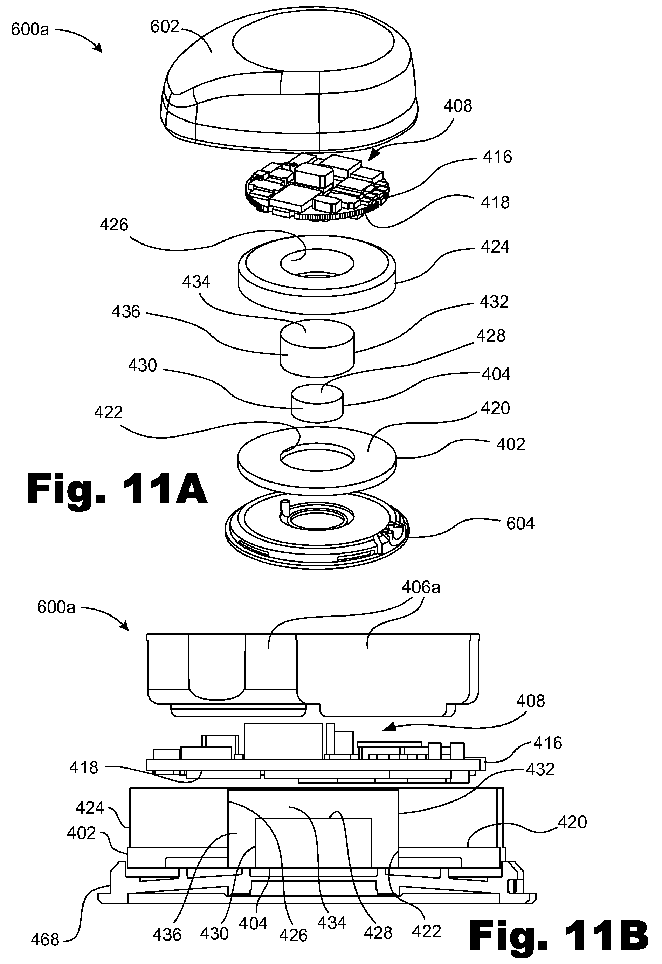

Turning to FIGS. 11A and 11B, the integrated speech processor headpiece 600a is substantially similar to the integrated speech processor headpiece 600. Here, however, the batteries 406a are located above the electronic circuitry 408 (such as the sound processor 106, or at least a portion thereof) carried on the circuit board 416 and the headpiece is configured to direct magnetic flux away from electronic circuitry 408. The magnetic flux direction aspects associated with the integrated speech processor headpiece 600a are applicable to the other integrated speech processor headpieces described herein.

In the illustrated embodiment, the bottom surface 418 of the circuit board 416 may face generally towards the headpiece base plate 604. The electronic circuitry 408 may be configured to direct the implantable cochlear stimulator 110 to generate and apply electrical stimulation to one or more stimulation sites within the cochlea of a patient by transmitting control parameters (including, but not limited to, stimulation parameters) to the implantable cochlear stimulation device 104 via communications link 118. The electronic circuitry 408 may additionally or alternatively be configured to transmit power to the implantable cochlear stimulation device 104 and may be configured to receive data from the cochlear stimulation device 104.

The induction coil 402 in the exemplary headpiece 600a illustrated in FIGS. 11A and 11B is disposed below the bottom surface 418 of the circuit board 416. The induction coil 402 may include a metallic wire or tube wound in a coiled configuration. In some examples, the induction coil 402 may include a coiled wire arranged in a generally disc-shaped and/or annular-shaped holder. It will be recognized that the induction coil 402 may have any suitable size and shape as may serve a particular application. The induction coil 402 may have a top surface 420 and an interior radial surface 422. The induction coil 402 may be seated in the headpiece base plate 604. Accordingly, the induction coil 402 may be in close proximity to the head of the patient when the headpiece base plate 604 is adjacent to the head.

With respect to magnetic flux direction, the headpiece 600a includes a telemetry flux guide 424 positioned or disposed between the circuit board 416 and the induction coil 402. The telemetry flux guide 424 may have a generally annular shape with an inner radial surface 426 defining an aperture extending through a central portion of the telemetry flux guide 424. The telemetry flux guide may be adjacent to the bottom surface 418 of the circuit board 416 and the top surface 420 of the induction coil 402.

The telemetry flux guide 424 may include any material suitable for directing magnetic flux away from the circuit board 416. For example, the telemetry flux guide 424 may include a material having a relatively high resistivity that provides a low reluctance path for magnetic flux of the telemetry magnetic field. Additionally, the telemetry flux guide 424 may include a powdered material, such as a powdered metallic material having a relatively small particle size, in order to prevent the generation of eddy current in the telemetry flux guide 424. For example, eddy currents might be generated in a solid conductive material (as opposed to a powdered conductive material) in the presence of the telemetry magnetic field since the telemetry magnetic field is a changing magnetic field generated by an alternating current passing through the induction coil 402.

The powdered material in the exemplary telemetry flux guide 424 may be held together using any suitable material, such as a polymer material. In some examples, the powdered metallic material may include iron and/or other ferrite materials. Additionally, the telemetry flux guide 424 may be suitable for frequencies of telemetry signals generated and/or received by the induction coil 402, such as, for example, an approximately 49 MHz telemetry signal and/or an approximately 10.7 MHz telemetry signal. A telemetry flux guide 424 including a material having a relative permeability (*i.e.,* the ratio of the permeability of the alloy to the permeability of free-space) of approximately 9 may be suitable for a frequency range that includes 49 MHz and 10.7 MHz telemetry signals. However, it will be recognized that the telemetry flux guide 424 may have any other suitable relative permeability value as may serve a particular application. Additionally or alternatively, telemetry flux guide 424 may have a relatively high resistivity and a relatively small particle size in order to facilitate redirection of magnetic flux while minimizing the generation of eddy currents in the telemetry flux guide 424.

The telemetry flux guide 424 is positioned and configured to direct magnetic flux of the magnetic field generated by the induction coil 402 away from the circuit board 416, as will be described in greater detail below. For example, a telemetry magnetic field may generated by the induction coil 402 to transmit a telemetry signal. Magnetic flux of the telemetry magnetic field is directed away from the circuit board 416 by the telemetry flux guide 424, thereby protecting the electronic circuitry 408 on the circuit board 416 from the telemetry magnetic field. By directing the magnetic flux in the telemetry magnetic field away from the circuit board 416, energy losses from the induction coil 402 to the electronic circuitry 408 via the telemetry magnetic field may be minimized, thereby extending the life of batteries used to provide power to one or more components of the cochlear implant system.

The headpiece 600a includes a retention magnet 404 disposed between the circuit board 416 and the headpiece base plate 604. The retention magnet 404 may have a top surface 428 generally facing the bottom surface 418 of the circuit board 416. The retention magnet 404 may additionally have an outer radial surface 430. In some embodiments, the retention magnet 404 may be positioned in the headpiece 600a such that the retention magnet 404 is at least partially surrounded by the induction coil 402 and/or the telemetry flux guide 424. Accordingly, the outer radial surface 430 of the retention magnet 404 may generally face the inner radial surface 422 of the induction coil 402 and/or the inner radial surface 426 of the telemetry flux guide 424. For example, the retention magnet 404 may be positioned in an aperture defined by the inner radial surface 426 extending through the telemetry flux guide 424.

The retention magnet 404 is configured to produce a retention magnetic field for securing one or more components of a cochlear implant system the head of the patient. For example, the retention magnet 404 may be disposed adjacent to the base plate 604 such that the retention magnet 404 is in close proximity to the head of the patient when the base plate 604 is adjacent to the head.

As noted above, the exemplary cochlear stimulation device 104 (FIG. 5) in the cochlear implant system 100 may include a magnet 123 configured to magnetically couple with the retention magnet 404. Accordingly, when the base plate 604 is positioned adjacent to the head of the patient near the coil 122, the retention magnet 404 may be magnetically coupled to the magnet of the cochlear stimulation portion 104, thereby securing and/or orienting the headpiece 600a on the head.

The headpiece 600a includes a retention flux guide 432 positioned between the circuit board 416 and the retention magnet 404. The retention flux guide 432 may at least partially surround the retention magnet 404. As illustrated in FIGS. 11A and 11 B, a top wall 434 of the retention flux guide 432 is disposed in between and adjacent to the bottom surface 418 of the circuit board 416 and the top surface 428 of the retention magnet 404. Additionally, a side wall 436 of the retention flux guide 432 may at least partially surround the outer radial surface 430 of the retention magnet 404. The side wall 436 of the retention flux guide 432 may be adjacent to the outer radial surface 430 of the retention magnet 404, the inner radial surface 422 of the induction coil 402, and/or the inner radial surface 426 of the telemetry flux guide 424.

The retention flux guide 432 may include any material suitable for redirecting magnetic flux associated with a magnetic field produced by the retention magnet 404. For example, the retention flux guide 432 may include a material having a relatively high permeability. In some examples, the retention flux guide 432 may include a metallic material, such as a mu-metal alloy comprising nickel and iron. A relatively high permeability mu-metal alloy may have a relative permeability between approximately 60,000 and 300,000. For example, a mu-metal alloy may have a relative permeability of approximately 100,000. A high-permeability mu-metal alloy may include any suitable ratio of nickel and iron, such as, for example, a ratio of approximately 80% nickel and 20% iron. It will be recognized that the retention flux guide 432 may alternatively include any suitable material having any suitable relative permeability.

The retention flux guide 432 is configured to direct magnetic flux of a retention magnetic field surrounding the retention magnet 404 away from the circuit board 416 and away from the telemetry flux guide 424. Accordingly, magnetic flux from the retention magnet 404 is directed away from the circuit board 416, thereby protecting the electronic circuitry 408 on the circuit board 416 from the retention magnetic field.

As mentioned, the retention flux guide 432 also directs magnetic flux of the retention magnetic field away from the telemetry flux guide 424, thereby preventing saturation of powdered metallic material in the telemetry flux guide 424 with magnetic flux from the retention magnet. Magnetic flux from the retention magnet 404 may significantly reduce the relative permeability of the powdered metallic material in the telemetry flux guide 424, reducing the effectiveness of the telemetry flux guide 424 in directing magnetic flux from the induction coil 402 away from the circuit board 416. Accordingly, the retention flux guide 432 directs magnetic flux of the retention magnetic field away from the telemetry flux guide 424, thereby preventing magnetic flux from saturating the telemetry flux guide 424.

By directing magnetic flux away from the electronic circuitry 408 in the circuit board 416, the telemetry flux guide 424 and the retention flux guide 432 enable the induction coil 402 and the retention magnet 404 to be located within the headpiece 600a in relatively close proximity to the circuit board 416. Accordingly, the integrated speech processor headpiece 600a may be made more compact by consolidating electronic and magnetic field emitting components within the headpiece, as illustrated in FIGS. 11A and 11 B. This increases the ease of use and comfort for a patient using the cochlear implant system in comparison to a larger integrated unit or a conventional cochlear implant systems (e.g. a body worn system or BTE system) in which electronic circuitry is separated from the headpiece.

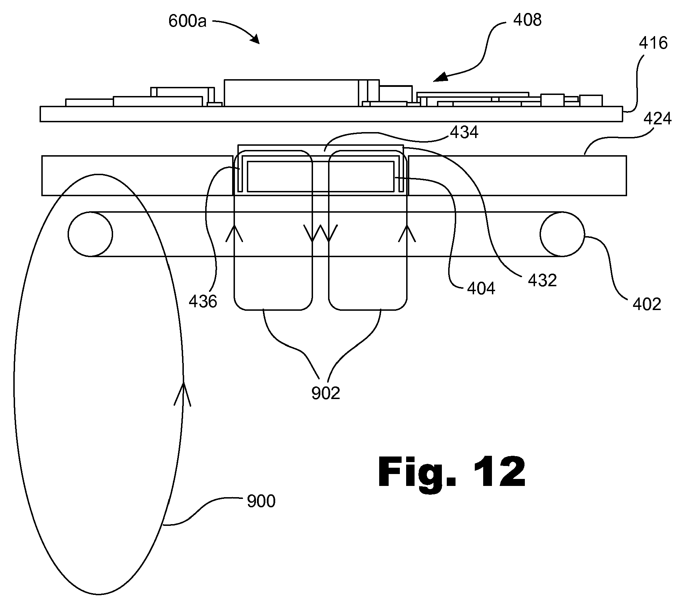

FIG. 12 illustrates magnetic flux surrounding an induction coil 402 and a retention magnet 404 in the headpiece 600a in accordance with the present invention. As illustrated in FIG. 12, magnetic flux 900 may surround the induction coil 402. The magnetic flux 900 is represented as a flux path surrounding the induction coil 402. Additionally, magnetic flux 902 may pass through and surround retention magnet 404. The magnetic flux 902 is represented as flux paths surrounding and passing through the retention magnet 404. It will be recognized that additional flux paths other than those illustrated in FIG. 12 may be associated with the telemetry magnetic field surrounding the induction coil 402 and the retention magnetic field surrounding the retention magnet 404.

The telemetry flux guide 424 may provide a low reluctance path for the magnetic flux 900 surrounding the induction coil 402. As illustrated in FIG. 12, the path of the magnetic flux 900 may be directed through the telemetry flux guide 424 such that a path of the magnetic flux 900 between the induction coil 402 and the circuit board 416 may be shortened. The magnetic flux 900 of the telemetry magnetic flux field surrounding the induction coil 402 may therefore be redirected by the telemetry flux guide 424 such that the magnetic flux 900 is substantially prevented from reaching the circuit board 416, thereby reducing or eliminating magnetic flux passing through the electronic circuitry 408.

The retention flux guide 432 may provide a high permeability path for the magnetic flux 902 surrounding and passing through the retention magnet 404. As illustrated in FIG. 12, the path of the magnetic flux 902 may be directed through the retention flux guide 432 such that a path of the magnetic flux 902 passes generally through and/or along the top wall 434 and/or the side wall 436 of the retention flux guide 432. Accordingly, a path of the magnetic flux 902 between the induction coil 402 and the circuit board 416 may be shortened. Similarly, a path of the magnetic flux 902 between the retention magnet 404 and the telemetry flux guide 424 may be shortened. The magnetic flux 902 of the retention magnetic flux field surrounding and passing through the retention magnet 404 may therefore be redirected by the retention flux guide 432 such that the magnetic flux 902 is substantially prevented from reaching the circuit board 416 and the telemetry flux guide 424, thereby reducing or eliminating magnetic flux from the retention magnet passing through the electronic circuitry 408 and the telemetry flux guide 424.

In sum, an integrated headpiece combines the external components of the cochlear implant system into a single unit that is worn directly over the surgically implanted receiver. This eliminates the need for a separate body worn processor or BTE processor and the connecting cable. Consequently, the integrated headpiece reduces the complexity of wearing and using a cochlear implant. The integrated headpiece eliminates the need to route a cable through clothing or hair and the possibility of snagging a cable. Additionally, the integrated headpiece can be more robust, and significantly less visually intrusive than processors of conventional cochlear implant systems.

The preceding description has been presented only to illustrate and describe embodiments and examples of the principles described. This description is not intended to be exhaustive or to limit these principles to any precise form disclosed. Many modifications and variations are possible within the scope of the invention as defined by the appended claim.

## Claims

1. An integrated headpiece for a cochlear implant system, comprising:
a microphone (108, 410) for outputting an audio signal;
a circuit board (416) carrying signal processing electronics (106, 408) for processing the audio signal; and
a transmitter comprising an induction coil (120, 402, 504) for transmitting a processed audio signal received from the electronics to an implanted receiver by generating a telemetry magnetic field, the implanted receiver sending the processed audio signal to an implanted processor, the implanted processor selectively energizing an electrode array having multiple stimulating electrodes; the integrated headpiece being configured as a stand alone, head-mounted external component which provides external functionality for operation of the cochlear implant system;
a retention magnet (404) configured to produce a retention magnetic field for securing the integrated headpiece to a head of the patient;
**characterised by**:
a telemetry flux guide (424) positioned between the induction coil and the circuit board, the telemetry flux guide being configured to direct magnetic flux of the telemetry magnetic field away from the circuit board; and
a retention flux guide (432) positioned between the retention magnet and the circuit board, and between the retention magnet and the telemetry flux guide, the retention flux guide being configured to direct magnetic flux of the retention magnetic field away from the circuit board and away from the telemetry flux guide;
wherein all of the microphone, circuit board, retention magnet, telemetry flux guide, retention flux guide and transmitter are disposed in a common rigid housing (414, 502, 602, 802/804) of the integrated headpiece.

2. The integrated headpiece of claim 1, wherein the integrated headpiece further comprises a power source (406), the power source supplying electrical power to the microphone, signal processing electronics and transmitter.

3. The integrated headpiece of claim 1, wherein the headpiece is further configured to receive an assistive listening device cap, the assistive listening device cap being magnetically attached to the headpiece and providing additional functionality to the headpiece.

4. The integrated headpiece of claim 1, wherein at least a portion of the induction coil (504) extends beyond a main portion of the integrated headpiece, thereby creating an aperture between the at least a portion of the induction coil and the main portion of the integrated headpiece.

5. The integrated headpiece of claim 1, wherein the integrated headpiece is waterproof and/or has no external electrical contacts.

6. The integrated headpiece of claim 1, wherein the integrated headpiece further comprises at least one visual indicator and/or at least one external switch (506).

7. The integrated headpiece of claim 1, wherein the integrated headpiece further comprises a receiver (412).

8. A system of integrated headpieces for a cochlear implant system, comprising:
a plurality of integrated headpieces (102, 400, 500, 600, 600a, 700) in a set, wherein each headpiece in the set is a headpiece according to claim 1.

9. The system of claim 8, wherein one of the integrated headpieces in the set comprises a receiver (412) for receiving an electronic signal from an external device.

## Patentansprüche

1. Integriertes Kopfstück für ein Cochlea-Implantat-System mit:
einem Mikrofon (108, 410) zum Ausgeben eines Audiosignals;
einer Leiterplatte (416), die eine Signalverarbeitungselektronik (106, 408) zum Verarbeiten des Audiosignals trägt;
einem Sender mit einer Induktionsspule (120, 402, 504) zum Senden eines verarbeiteten Audiosignals, welches von der Elektronik empfangen wurde, zu einem implantierten Empfänger mittels Erzeugung eines Telemetrie-Magnetfelds, wobei der implantierte Empfänger das verarbeitete Audiosignal an einen implantierten Prozessor sendet, der ein Elektrodenfeld mit mehreren Stimulationselektroden selektiv mit Energie beaufschlägt; wobei das integrierte Kopfstück als eigenständige, am Kopf montierte externe Komponente konfiguriert ist, die für eine externe Funktionalität zum Betreiben des Cochlea-Implantat-Systems sorgt; und
einem Haltemagneten (404), der für das Erzeugen eines Haltemagnetfelds zum Befestigen des integrierten Kopfstücks am Kopf des Patienten konfiguriert ist;
**gekennzeichnet durch**:
eine Telemetrieflussführung (424), die zwischen der Induktionsspule und der Leiterplatte angeordnet ist und konfiguriert ist, um magnetischen Fluss des Telemetrie-Magnetfelds von der Leiterplatte weg zu führen; und
eine Halteflussführung (432), die zwischen dem Haltemagneten und der Leiterplatte und zwischen dem Haltemagneten und der Telemetrieflussführung angeordnet ist und konfiguriert ist, um magnetischen Fluss des Haltemagnetfelds von der Leiterplatte und von der Telemetrieflussführung weg zu führen;
wobei das Mikrofon, die Leiterplatte, der Haltemagnet, die Telemetrieflussführung, die Halteflussführung und der Sender in einem gemeinsamen starren Gehäuse (414, 502, 602, 802/804) des integrierten Kopfstücks angeordnet sind.

2. Integriertes Kopfstück gemäß Anspruch 1, wobei das integrierte Kopfstück ferner eine Stromquelle (406) aufweist, welche das Mikrofon, die Signalverarbeitungselektronik und den Sender mit Strom versorgt,

3. Integriertes Kopfstück gemäß Anspruch 1, wobei das Kopfstück ferner konfiguriert ist, um eine Hilfshörgerätkappe zu empfangen, welche magnetisch an dem Kopfstück befestigt ist und dem Kopfstück zusätzliche Funktionalität verleiht.

4. Integriertes Kopfstück gemäß Anspruch 1, wobei sich mindestens ein Teil der Induktionsspule (504) über einen Hauptabschnitt des integrierten Kopfstücks hinaus erstreckt und dadurch eine Öffnung zwischen dem mindestens einen Teil der Induktionsspule und dem Hauptabschnitt des integrierten Kopfstücks erzeugt.

5. Integriertes Kopfstück gemäß Anspruch 1, wobei das integrierte Kopfstück wasserdicht ist und/oder keine externen elektrischen Kontakte aufweist.

6. Integriertes Kopfstück gemäß Anspruch 1, wobei das integrierte Kopfstück mindestens einen visuellen Indikator und/oder mindestens einen externen Schalter (506) aufweist.

7. Integriertes Kopfstück gemäß Anspruch 1, wobei das integrierte Kopfstück ferner einen Empfänger (412) aufweist.

8. System von integrierten Kopfstücken für ein Cochlea-Implantat-System mit:
einer Mehrzahl von integrierten Kopfstücken (102, 400, 500, 600, 600a, 700) in einem Set, wobei jedes Kopfstück in dem Set ein Kopfstück gemäß Anspruch 1 ist.

9. System gemäß Anspruch 8, wobei eines der integrierten Kopfstücke in dem Set einen Empfänger (412) zum Empfangen eines elektronischen Signals von einem externen Gerät aufweist.

## Revendications

1. Élément de tête intégré pour un système d'implant cochléaire, l'élément de tête intégré comprenant :
un microphone (108, 410) servant à produire un signal audio ;
une carte de circuit imprimé (416) portant de l'électronique de traitement de signal (106, 408) servant à traiter le signal audio ; et
un émetteur comprenant une bobine d'induction (120, 402, 504) servant à émettre un signal audio traité reçu depuis l'électronique à destination d'un récepteur implanté en générant un champ magnétique de télémesure, le récepteur implanté envoyant le signal audio traité à un processeur implanté, le processeur implanté excitant de façon sélective un groupement d'électrodes comportant plusieurs électrodes de stimulation ; l'élément de tête intégré étant configuré comme un composant autonome externe monté sur la tête qui procure une fonctionnalité externe pour le fonctionnement du système d'implant cochléaire ;
un aimant de retenue (404) configuré pour produire un champ magnétique de retenue servant à assujettir l'élément de tête intégré à la tête du patient ;
l'élément de tête intégré étant **caractérisé par** :
un guide de flux de télémesure (424) placé entre la bobine d'induction et la carte de circuit imprimé, le guide de flux de télémesure étant configuré pour diriger le flux magnétique du champ magnétique de télémesure à l'écart de la carte de circuit imprimé ; et
un guide de flux de retenue (432) placé entre l'aimant de retenue et la carte de circuit imprimé, et entre l'aimant de retenue et le guide de flux de télémesure, le guide de flux de retenue étant configuré pour diriger le flux magnétique du champ magnétique de retenue à l'écart de la carte de circuit imprimé et à l'écart du guide de flux de télémesure ;
dans lequel le microphone, à carte de circuit imprimé, l'aimant de retenue, le guide de flux de télémesure, le guide de flux de retenue et l'émetteur sont tous mis en place dans un boîtier rigide commun (414, 502, 602, 802/804) de l'élément de tête intégré.

2. Élément de tête intégré selon la revendication 1, lequel élément de tête intégré comprend en outre une source d'énergie (406), la source d'énergie alimentant en énergie électrique le microphone, l'électronique de traitement de signal et l'émetteur.

3. Élément de tête intégré selon la revendication 1, lequel élément de tête est configuré en outre pour recevoir un embout de dispositif d'écoute d'assistance, l'embout de dispositif d'écoute d'assistance s'attachant magnétiquement à l'élément de tête et procurant une fonctionnalité supplémentaire à l'élément de tête.

4. Élément de tête intégré selon la revendication 1, dans lequel une partie au moins de la bobine d'induction (504) s'étend au-delà d'une partie principale de l'élément de tête intégré de manière à créer une ouverture entre ladite partie au moins de la bobine d'induction et la partie principale de l'élément de tête intégré.

5. Élément de tête intégré selon la revendication 1, lequel élément de tête intégré est étanche et/ou ne comporte pas de contacts électriques externes.

6. Élément de tête intégré selon la revendication 1, lequel élément de tête intégré comprend en outre au moins un indicateur visuel et/ou au moins un commutateur externe (506).

7. Élément de tête intégré selon la revendication 1, lequel élément de tête intégré comprend en outre un récepteur (412).

8. Système d'éléments de tête intégrés pour un système d'implant cochléaire, le système comprenant :
une pluralité d'éléments de tête intégrés (102, 400, 500, 600, 600a, 700) dans un jeu, chaque élément de tête dans le jeu étant un élément de tête selon la revendication 1.

9. Système selon la revendication 8, dans lequel l'un des éléments de tête intégrés dans le jeu comprend un récepteur (412) servant à recevoir un signal électronique depuis un dispositif externe.
